# EUROPEAN PATENT SPECIFICATION

(11) **EP 2 355 887 B1**
(45) Date of publication and mention of the grant of the patent: **02.08.2017**
(21) Application number: 09828101.7
(22) Date of filing: 17.11.2009
(51) Int. Cl.: A61B 17/20, A61K 9/00, A61M 37/00, A61B 5/15

(54) **HOLLOW MICRONEEDLE ARRAY**
HOHLMIKRONADELFELD
ENSEMBLE DE MICROAIGUILLES CREUSES

(30) Priority: 18.11.2008 US 115840 P
(43) Date of publication of application: 17.08.2011
(73) Proprietor: 3M Innovative Properties Company, St. Paul, MN 55133-3427 (US)
(72) Inventor: BURTON, Scott A.,, Saint Paul, Minnesota 55133-3427 (US); FREDERICKSON, Franklyn L., Esko, Minnesota 55733 (US); HANSEN, Kristen J., Saint Paul, Minnesota 55133-3427 (US); SIMMERS, Ryan Patrick,, Saint Paul, Minnesota 55133-3427 (US); FENN, Percy T.,, Saint Paul, Minnesota 55133-3427 (US); MOECKLY, Craig S.,, Saint Paul, Minnesota 55133-3427 (US)
(74) Representative: Vossius & Partner Patentanwälte Rechtsanwälte mbB
(86) International application number: PCT/US2009/064742
(87) International publication number: WO 2010/059605

(56) References cited:
- WO-A1-2006/101459
- WO-A1-2008/053481
- WO-A2-03/074102
- US-A1- 2005 209 565

## Description

### Field

The present invention relates to hollow microneedle drug delivery devices.

### Background

Transdermal patches have long been used for the administration of small molecule lipophilic drugs that can be readily absorbed through the skin. This non-invasive delivery route is advantageous for the administration of many drugs incompatible with oral delivery, as it allows for direct absorption of the drug into the systemic circulation, bypassing both the digestive and hepatic portal systems which can dramatically reduce the bioavailability of many drugs. Transdermal delivery also overcomes many of the challenges associated with subcutaneous injection by greatly reducing patient discomfort, needle anxiety, risk of accidental injury to the administrator and issues surrounding sharps disposal.

Despite these many advantages, transdermal delivery of drugs is confined to classes of molecules compatible with absorption through the skin. Delivery of small molecule salts and therapeutic proteins are not typically viable with traditional transdermal delivery, as the skin provides an effective protective barrier to these molecules even in the presence of absorption-enhancing excipients.

Microneedle (including microblade) drug delivery devices have been proposed based on a wide variety of designs and materials. Some are solid, e.g., with drug coated thereon, and others are hollow, e.g., with drug delivered from a reservoir. Some are made of metal, whereas others are etched from silicon material, and still others are made of plastics such as polycarbonate.

The number, size, shape, and arrangement of the microneedles also varies considerably. Some have a single needle, while others, especially solid microneedles, have hundreds of needles per array. Most range in size from 100 microns to 2 mm.

Microneedles have shown promise for delivery drugs intradermally and transdermally, particularly where a relatively small quantity of drug is needed such as in the case of vaccines or potent drugs.

One of the desired benefits of microneedles is of course to replace, where appropriate, conventional hypodermic needles, which can cause anxiety and/or pain for many patients. There are also benefits to delivering some drugs, e.g., vaccines, into the skin rather than via intramuscular injection. However, microneedle delivery systems often have been seen as providing quite low rates of delivery, thus limiting the usefulness of such systems by requiring either small quantities of drug formulation to be used or long delivery times. For example, typical intradermal infusion using microneedles has been documented with slow infusion rates of less than 30mcL/hour, and low infusion volumes less than 200mcL. Some reports have also indicated significant pain if higher infusion rates are attempted.

WO 03/074102 discloses a device and a method for transporting fluids across biological barriers that enhances penetration of a biological barrier by the use of directional insertion, preferably with asymmetric microneedles and/or microneedles with sharp edges. Also disclosed is a device and a method which combine shallow penetration of hollow microneedles with jet injection via the microneedles to achieve a total liquid penetration depth greater than the mechanical penetration depth of the microneedles.

WO 2008/053481 discloses a microneedle array that includes a plurality of solid or hollow microneedles which are designed for delivery of an agent into skin for treating skin contour irregularities or skin disorders.

US 2005/209565 discloses microneedle arrays having a separation distances between microneedles in the range of 100-300 microns, and more preferably in the range of 100-200 microns.

### Summary

It has now been found that the number of microneedles used and their density per unit area can produce much larger rates of delivery with virtually no pain induced. This offers for the first time the prospect for using microneedle arrays to replace hypodermic injections for rapid, painless delivery of injectable drug formulations. The inventive microneedle array is a hollow microneedle array (11) for rapid, high-volume, intradermal infusion with minimal pain, the hollow microneedle array (11) comprising: an array of 10 to 30 hollow microneedles (14) having a length of greater than lOOum to less than 1mm; the hollow microneedle array (11) being adapted for pumping greater than 200 uL of fluid through the hollow microneedles at a rate of greater than 20 uL/min; and being characterized in that the microneedles (14) are spaced an average of at least 2 mm apart from each other.

In a preferred embodiment the hollow microneedle array has 13 to 20 microneedles (14).

In another preferred embodiment the hollow microneedles (14) have an average channel bore of 20 to 50 um2 cross-sectional area.

In a further preferred embodiment the microneedles (14) have a length of between 500 um and 750 um.

In a still further preferred embodiment the microneedles (14) have an exit hole located on a sidewall of each microneedle (14). In preferred configurations the microneedle arrays of the present invention can deliver up to 1mL or more of liquid formulation at the astonishingly high rate of 500uL/min. Thus, for example, in contrast to other reported microneedle arrays that only deliver 100uL at a slow rate of 10uL per hour (not per minute), the present microneedle arrays can delivery a full 1mL injection intradermally in about a minute or less.

A microneedle array according to the invention will generally have from 13 to 20 microneedles, with a spacing density of 30 to 50 microneedles per cm². In one example 18 microneedles are used.

The microneedles generally have a length of between 500 um and 750 um, and an average channel bore of 20 to 50 µm² cross-sectional area.

The hollow microneedle array of the invention can provide infusion whereby at least 750uL of fluid is pumped through the microneedles. The fluid may be pumped through the hollow microneedles at a rate of at least 400 uL/min. The back pressure during pumping is usually no greater than 25 psi (172.37 Kilopascal) and generally maintained at 20 psi (137.90 Kilopascal). The microneedles have an exit hole located on a sidewall of each microneedle.

The microneedles typically penetrate from 100 um to 400 um into the dermis (hence the depth of penetration is not the full height of the microneedles themselves).

Without wishing to be bound to any particular theory, many prior art microneedle arrays appear to use a large number of closely spaced microneedles, which may limit the volume and rate of fluid that can be accommodated within the dermal tissue. Trying to inject fluid rapidly with such devices may then either create undue back-pressure, fluid leakage back out of the skin during injection, needle array dislodgement, tissue doming, and/or significant pain.

As used herein, certain terms will be understood to have the meaning set forth below:
"Microneedle" refers to a specific microscopic structure associated with the array that is designed for piercing the stratum corneum to facilitate the transdermal delivery of therapeutic agents or the sampling of fluids through the skin. By way of example, microneedles can include needle or needle-like structures, including microblades, as well as other structures capable of piercing the stratum corneum.

The features and advantages of the present invention will be understood upon consideration of the detailed description of the preferred embodiment as well as the appended claims. These and other features and advantages of the invention may be described below in connection with various illustrative embodiments of the invention. The above summary of the present invention is not intended to describe each disclosed embodiment or every implementation of the present invention. The Figures and the detailed description which follow more particularly exemplify illustrative embodiments.

### Brief Description of the Drawings

Preferred embodiments of the invention will now be described in greater detail below with reference to the attached drawings, wherein:
Figures 1A and B are a perspective view of a microneedle array embodiment, also showing a closer view of an individual hollow microneedle.
Figures 2A and B show images of hairless guinea pig skin after hollow microneedle patch removal with staining.
Figures 3A and B show images of a microneedle infusion site showing methylene blue
Figure 4 shows a comparative graph of naloxone blood levels versus time by delivery route.
Figure 5 plots pain of infusion versus certain infusion categories.
Figure 6 plots maximum infusion pressure versus certain infusion categories.
Figure 7 plots maximum infusion rate versus certain infusion categories.
Figure 8 plots infusion volume versus certain infusion categories.
Figure 9 plots pain of infusion versus maximum infusion pressure.
Figure 10 plots pain of infusion versus maximum infusion rate.
Figure 11 plots pain of infusion versus infusion volume.

While the above-identified drawing figures set forth several embodiments of the invention, other embodiments are also contemplated, as noted in the discussion. In all cases, this disclosure presents the invention by way of representation and not limitation. It should be understood that numerous other modifications and embodiments can be devised by those skilled in the art, which fall within the scope of the principles of the invention. The figures may not be drawn to scale. Like reference numbers have been used throughout the figures to denote like parts.

### Detailed Description

The invention will now be described with reference to the following non-limiting embodiment.

### Microneedle Array

A microneedle device 10 has a microneedles array 11 comprising a substrate 12 from which extend a plurality of eighteen microneedles 14. Each microneedle 14 has a height of approximately 500 µm from its base 16 to its tip 18. A hollow channel (not shown) extends through the substrate 12 and microneedle 14, exiting at a channel opening 20 near the tip of the microneedle. This allows fluid communication from the back of the array (e.g., from a reservoir, not shown) through each microneedle 14. The channel runs along a central axis of the microneedle 14, but exits similar to a hypodermic needle on a sloping side-wall of the microneedle to help prevent blockage by tissue upon insertion. The channel has an average cross-sectional area about 20-50 µm².

The microneedles 14 are spaced apart so that the distance d between adjacent microneedles 14 is 2 mm. The disk shaped substrate 12 has an area of about 1.27 cm² and the microneedles 14 are spread out over an area of about .42 cm² as measured using the perimeter of the outermost rows of microneedles 14. This gives a microneedle density of about 14 microneedles/cm².

The microneedle array 11 is made by thermocycled injection molding of a polymer such as medical grade polycarbonate, followed by laser drilling to form the channel of the microneedle.

An array rim structure 22 is used for attaching to the microneedle substrate 12 a backing member (not shown) that incorporates an adhesive disk (not shown) (3M 1513 Medical Tape, 3M Corp, St. Paul MN) that will extent outward from the perimeter 24 of the substrate 12 to secure the hollow microneedle array 11 to the skin during infusion. The skin contacting surface of the entire microneedle device 10 including an adhesive ring will be about 5.5 cm².

The microneedle device 10 is typically applied to the skin using an external applicator (not shown). The applicator is designed, e.g., using a spring mechanism, to achieve a desired velocity so the microneedles will penetrate into the skin rather then merely deforming the skin. Once applied, the adhesive ring secures the microneedle device against the skin. Various applicator devices are disclosed in, for example, WO2005/123173, WO2006/055802, WO2006/05579, WO2006/055771, WO2006/108185, WO2007/002521, and WO2007/002522.

Fluid to be delivered through the microneedle array can be contained in a reservoir (not shown) containing the fluid or by having the fluid pumped from an external source such as a syringe or other container that may be connected by, e.g., tubing or using a luer connector. Drug can be dissolved or suspended in the formulation, and typical formulations are those of the type that can be injected from a hypodermic needle.

Any substance that can be formulated and delivered via hypodermic injection may be used, including any pharmaceutical, nutraceutical, cosmaceutical, diagnostic, and therapeutic agents (collectively referred to herein as "drug" for convenience). Examples of drugs that may be useful in injectable formulations with the present invention include but are not limited to ACTH (e.g. corticotropin injection), luteinizing hormone-releasing hormone (e.g., Gonadorelin Hydrochloride), growth hormone-releasing hormone (e.g., Sermorelin Acetate), cholecystokinin (Sincalide), parathyroid hormone and fragments thereof (e.g. Teriparatide Acetate), thyroid releasing hormone and analogs thereof (e.g. protirelin), secretin and the like, Alpha-1 anti-trypsin, Anti-Angiogenesis agents, Antisense, butorphanol, Calcitonin and analogs, Ceredase, COX-II inhibitors, dermatological agents, dihydroergotamine, Dopamine agonists and antagonists, Enkephalins and other opioid peptides, Epidermal growth factors, Erythropoietin and analogs, Follicle stimulating hormone, G-CSF, Glucagon, GM-CSF, granisetron, Growth hormone and analogs (including growth hormone releasing hormone), Growth hormone antagonists, Hirudin and Hirudin analogs such as Hirulog, IgE suppressors, Insulin, insulinotropin and analogs, Insulin-like growth factors, Interferons, Interleukins, Luteinizing hormone, Luteinizing hormone releasing hormone and analogs, Heparins, Low molecular weight heparins and other natural, modified, or synethetic glycoaminoglycans, M-CSF, metoclopramide, Midazolam, Monoclonal antibodies, Peglyated antibodies, Pegylated proteins or any proteins modified with hydrophilic or hydrophobic polymers or additional functional groups, Fusion proteins, Single chain antibody fragments or the same with any combination of attached proteins, macromolecules, or additional functional groups thereof, Narcotic analgesics, nicotine, Non-steroid anti-inflammatory agents, Oligosaccharides, ondansetron, Parathyroid hormone and analogs, Parathyroid hormone antagonists, Prostaglandin antagonists, Prostaglandins, Recombinant soluble receptors, scopolamine, Serotonin agonists and antagonists, Sildenafil, Terbutaline, Thrombolytics, Tissue plasminogen activators, TNF-, and TNF-antagonist, the vaccines, with or without carriers/adjuvants, including prophylactics and therapeutic antigens (including but not limited to subunit protein, peptide and polysaccharide, polysaccharide conjugates, toxoids, genetic based vaccines, live attenuated, reassortant, inactivated, whole cells, viral and bacterial vectors) in connection with, addiction, arthritis, cholera, cocaine addiction, diphtheria, tetanus, HIB, Lyme disease, meningococcus, measles, mumps, rubella, varicella, yellow fever, Respiratory syncytial virus, tick borne Japanese encephalitis, pneumococcus, streptococcus, typhoid, influenza, hepatitis, including hepatitis A, B, C and E, otitis media, rabies, polio, HIV, parainfluenza, rotavirus, Epstein Barr Virsu, CMV, chlamydia, non-typeable haemophilus, moraxella catarrhalis, human papilloma virus, tuberculosis including BCG, gonorrhoea, asthma, atherosclerosis malaria, E-coli, Alzheimer's Disesase, H. Pylori, salmonella, diabetes, cancer, herpes simplex, human papilloma and the like other substances including all of the major therapeutics such as agents for the common cold, Anti-addiction, anti-allergy, anti-emetics, anti-obesity, antiosteoporeteic, anti-infectives, analgesics, anesthetics, anorexics, antiarthritics, antiasthmatic agents, anticonvulsants, anti-depressants, antidiabetic agents, antihistamines, anti-inflammatory agents, antimigraine preparations, antimotion sickness preparations, antinauseants, antineoplastics, antiparkinsonism drugs, antipruritics, antipsychotics, antipyretics, anticholinergics, benzodiazepine antagonists, vasodilators, including general, coronary, peripheral and cerebral, bone stimulating agents, central nervous system stimulants, hormones, hypnotics, immunosuppressives, muscle relaxants, parasympatholytics, parasympathomimetrics, prostaglandins, proteins, peptides, polypeptides and other macromolecules, psychostimulants, sedatives, and sexual hypofunction and tranquilizers.

It will be understood that a wide range of hollow microneedle shapes can be used, such as cone shaped, cylindrical, pyramidal, truncated, asymmetrical, and combinations thereof. Various materials can also be used, such as polymers, metals, and silicon-based, and can be manufactured in any suitable way, such as injection molding, stamping, and using photolithography. The arrangement of the microneedles on the substrate can be of any pattern, such as random, polygonal, square, and circular (as viewed facing to the skin-contacting surface of the array).

In addition to the above description, the following patent documents disclose microneedle devices, materials, fabrication, applicators, and uses that are useful or adaptable for use according to the present invention: US 6,881,203; US 6,908,453; US 2005-0261631; WO2005/065765; WO2005/082596; WO2006/062974; WO2006/135794; US2006/048640; US provisional application 60/793611; US2007/064789; WO2006/062848; WO2007/002523; and US provisional application 60/793564.

### Experimental

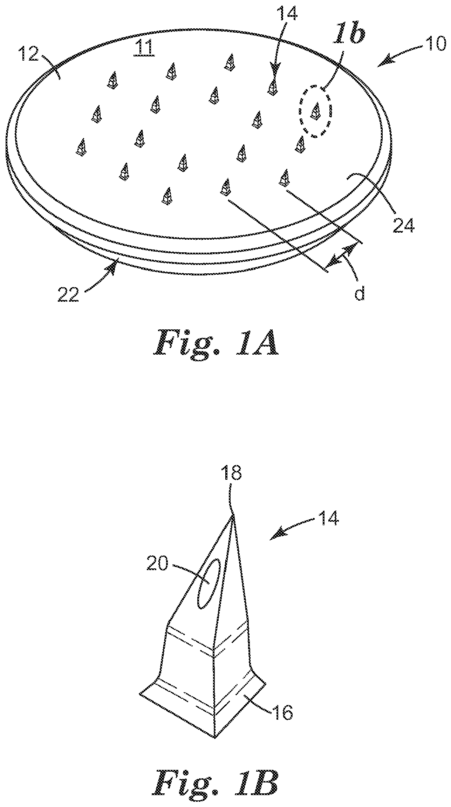

The microneedle array device as described above in connection with the Figure 1A was used for the following experiments and examples.

### Animal Models and Skin Preparation

### Hairless Guinea Pigs (HGP)

Male HGPs were ordered from Charles River Laboratories (Wilmington, MA) under a 3M IACUC-approved animal use application and used according to that protocol. All animals used in this study weighed 0.8-1kg.

### Domestic Pigs

Testing was conducted on female domestic pigs approximately 6-18 weeks old and weighing approximately 10-30kg, and obtained under a 3M IACUC-approved animal use application. During infusion and throughout the studies, the pigs were maintained under anesthesia with isoflurane (2-5%) oxygen mix. The upper portion of the pig's hip was shaved first using a surgical clipper (clip blade #50) and then with a Schick 3 razor using a small amount of Gillette Foam shaving cream. After shaving, the site was rinsed with water, patted dry and then wiped with isopropyl alcohol (Phoenix Pharmaceutical, Inc., St. Joseph, MO).

### Serum Naloxone Determination

At each time point, 1.5-2mL of whole blood was collected from the ear vein of the pig using a Vacutainer Collections Set (Becton Dickenson & Co., Franklin Lakes, NJ). The blood was allowed to set at room temperature for at least 30 minutes prior to being centrifuged at 1500 rpm for 10 minutes. After centrifugation, the serum was separated from the whole blood and stored cold until extraction.

Room temperature serum samples were prepared using solid phase extraction cartridges (Phenomenex, Torrance, CA). Cartridges were conditioned with methanol (EMD Chemicals, Inc, Gibbstown, NJ) and equilibrated with reagent grade water before loading with the serum samples. Serum was washed with 2mL of 5% methanol in reagent grade water and naloxone eluted with 100% methanol. The eluent was collected in a 14mL glass tube or a 16x100 mm tube and dried under 15psi of nitrogen in a 37C water bath.

Extracts were reconstituted with 5% acetonitrile/95% 0.1% formic acid (Alfa Aesar, Ward Hill, MA) in water, transferred to microcentrifuge tubes (Eppendorf, Westbury, NY) and centrifuged at 14000rpm for 10 minutes.

Extracts were quantitatively analyzed using LCMSMS. Separation was achieved using an Agilent Eclipse XDB-C18 column (Agilent Technologies, Wilmington, DE) in sequence with a Phenomenex C18 Guard Column (Phenomenex, Torrence, CA); the mobile phase was 0.1% formic acid and acetonitrile; the formic acid was ramped from 95% to 10% over 1 minute. A Sciex API3000 triple quad mass spectrometer (Applied Biosystems, Foster City, CA) running in positive ion mode using a Turbo IonSpray interface, was used to quantitatively monitor the product ions resulting from the following m/z transitions: 328.17 → 310.10 and 342.16 → 324.30. The linear range for naloxone was 0.1 to 100ng/mL evaluated using a 1/x curve weighting.

Various sizes of pigs were dosed, so to normalize blood naloxone levels with respect to pig weight, the blood naloxone levels were multiplied by a conversion factor of 62mL blood/kg of pig weight and then multiplied by the weight of the pig at dosing (kg). Final results are plotted as µg naloxone/pig.

### Depth of Penetration in HGPs and Pigs

Based on the technical literature, and considering the size of the microstructures, it was estimated that a force of 0.004-0.16 N per microstructure is required for penetration of the stratum corneum. S.P. David, B.J. Landis, Z.H. Adams, M.G. Allen, M.R. Prausnitz. Insertion of microneedles into skin: measurement and prediction of insertion force and needle fracture force. Journal of Biomechanics. 37:115-116 (2004). To ensure sufficient durability of the microstructures used herein, the array was pressed against a non-elastic surface; tip bending occurred when approximately 245 N of force was applied to the array.

With the exception that it contains no sweat glands, porcine skin is generally regarded as being similar to human skin in thickness, hair density and attachment to the underlying tissue. If the depth of the epidermis in the pig used in these studies is approximately similar to that found in humans, depth of penetration data indicate that the likely depth of infusion for the hollow microneedle devices used herein (see Figure 1A) is 180-280µm (average 250µm), a depth that could correspond to either the dermis or the epidermis which may affect the magnitude of back pressure encountered during infusion. It will thus be understood that although the microneedle height was about 500 µm, the actual depth of penetration was about half of that.

The depth of penetration (DOP) experiments were completed in both HGPs and in domestic pigs; the results are summarized in Table I.

**Table I. Summary of DOP Data collected on HGPs and Domestic Pigs**

| | **DOP in HGPs** | **DOP in Pig** |
|---|---|---|
| **Number of applications*** | 6 | 6 |
| **Average (µm)** | 210µm | 250µm |
| **Standard Deviation (µm)** | 30µm | 40µm |
| **%RSD** | 15% | 16% |

| | | |
|---|---|---|
| * each application consists of 1 array with 18 measured microstructures | | |

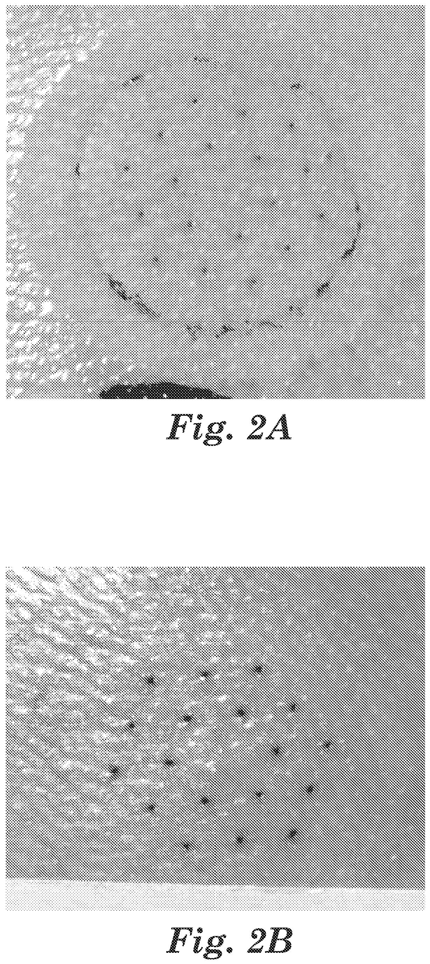

Fracturing of the microstructures was not observed in the force testing experiment nor were any broken needles observed following DOP testing. Figures 2A and 2B show an application site on an HGP after patch removal. Figure 2A shows markings made by Rhodamine B dye that had been coated on the microneedles prior to application. Figure 2B shows markings made by staining with methylene blue after a microneedle array was removed. Penetration of the stratum corneum by each of the 18 microstructures is evident from the pattern of methylene blue dots in Figure 2B. No blood was observed during or after application.

In swine, several infusions of up to 1mL were conducted using a sterile 5% dextrose or 0.001% methylene blue solution. Once the formulation had been delivered, the device was allowed to stay in place for up to 10 minutes while the back pressure on the system returned to pre-infusion levels. Figure 2 shows the results of an 800µL intradermal infusion of a 0.001% methylene blue formulation into pig. The skin is dry to the touch after patch removal; the deep blue of the infused formulation provides a visual assessment of the treatment.

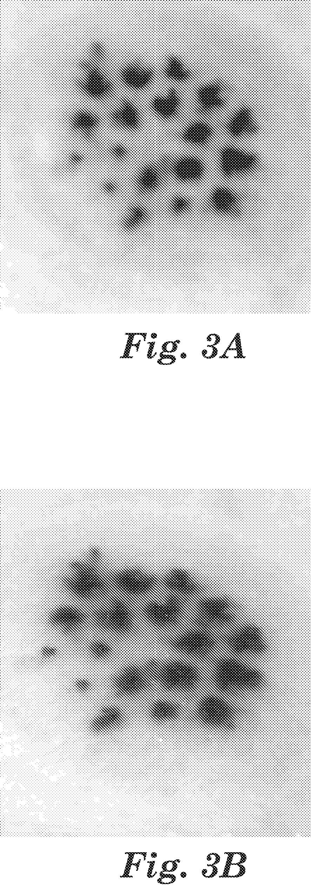

Figures 3A and 3B show images of intradermal infusion of a 0.05% methylene blue formulation in pig at T= 0 and T= 9 min, respectively, post-patch removal. The skin was dry to the touch.

Each blue spot on the skin corresponds to one of the eighteen hollow microstructures on the array. Although the dye appears somewhat smeared (diffused) after nine minutes, the blue stain remained, essentially unchanged 24 hours later although the wheal disappeared in under an hour. It is likely that the dye actually stained or precipitated in the tissue and, in this sense, is probably not an effective indicator of extended intradermal infusion patterns post infusion.

Upon removal of the hollow microneedle patch after infusion, a small amount (1-3µL) of formulation is typically observed on the surface of the skin. When this fluid is removed by gentle wiping with a tissue, no additional fluid is observed. A pinkish blotch, the size of the hollow microneedle array, is typically seen upon patch removal, but the blotch fades so as to become nearly indistinguishable within 5 minutes. A small dome, again approximately the size of the hollow microneedle array was observed on the pig skin as well. The dome yielded, but did not "leak", under gentle pressure. The dome was resolved, both visually and by touch, within 40 minutes of removing the application patch. Observations of the application site 24- and 48-hours post application showed no evidence of erythema or edema.

### Example 1. High Volume Dextrose Infusion in Pigs

High volume infusions have been demonstrated in domestic swine. Connected to the hollow microneedle array patch after application, the infusion system used with the swine employs standard medical equipment to provide delivery of the formulation. The hollow microneedle application patch is coupled to a Medfusion 3500 syringe pump (Smiths Medical, St. Paul, MN) via a commercial, pre-sterilized Polyethylene IV Extension Set (Vygon Corporation, Ecouen, France) that includes an in-line pre-sterilized, DTX Plus TNF-R pressure transducer (BD Infusion Therapy Systems, Inc, Sandy, UT). The Medfusion 3500 pump is commonly used in hospital settings and has pre-set safety stop features. Pressure readings were recorded at a rate of approximately one measurement every two seconds. A 5% Dextrose, USP, solution for injection (Baxter Healthcare, Deerfield, IL) was used for infusion as received. The 0.001% methylene blue solution was prepared using sterile water and was filtered prior to administration.

Testing was conducted on female domestic pigs approximately 6-18 weeks old and weighing approximately 10-30kg, and obtained under a 3M IACUC-approved animal use application. During infusion and throughout the studies, the pigs were maintained under anesthesia with isoflurane (2-5%) and an oxygen mix. The upper portion of the pig's hip was shaved first using a surgical clipper (clip blade #50) and then with a Schick 3 razor using a small amount of Gillette Foam shaving cream. After shaving, the site was rinsed with water, patted dry and then wiped with iso-propyl alcohol (Phoenix Pharmaceutical, Inc., St. Joseph, MO).

Up to 1mL of 5% dextrose in water or up to 425mcL of naloxone was delivered to the upper hip portion of the swine. Back pressure was monitored continually during the infusion to verify the absence of a leak in the infusion system. Typical infusion rate profiles utilized in the swine is provided in Table II, below.

**Table II. Summary of infusion conditions for 2 separate 1 mL infusions of dextrose into pig**

| **Infusion** | **Max Rate (µL/min)** | **Infusion Rate Program in µL/min (time)** |
|---|---|---|
| 1003µL dextrose | 50 | 10(5min), 20(7.5min), 30(10min), 40(7.5min), 50 (4min) |
| 1003µL dextrose | 75 | 10(1min), 25(2min), 50(4min), 75(approx 10min) |
| 425µL naloxone | 100 | 10(1min), 25(1min), 50(1min), 100(duration) |
| 330µL naloxone | 75 | 10(1min), 25(2min), 50(5min), 75(duration) |

After infusion, the hollow microneedle array was removed, leaving a small bleb under the skin. This bleb disappeared completely within 40 minutes. No site reaction was observed on the swine during observations through 48 hours post patch-removal.

Back pressure was monitored and recorded continuously during the dextrose and methylene blue infusions. The maximum back pressure measured, along with infusion conditions, for three infusions are provided in Table III.

**Table III. Summary of infusion conditions for 2 separate 1 mL infusions of dextrose into pig**

| **Infusion** | **Max Rate (µL/min)** | **Max Back Pressure (psi)** | **Infusion Rate Program in µL/min (time)** |
|---|---|---|---|
| 1003µL dextrose | 50 | 9.1 | 10(5min), 20(7.5min), 30(10min), 40 (7.5min), 50(4min) |
| 1003µL dextrose | 75 | 4.4 | 10(1min), 25(2min), 50(4min), 75(approx 10min) |
| 750µL methylene blue | 100 | 16.2 | 10(1min), 25(1min), 50(1min), 100 (duration) |

### Example 2. Naloxone Infusion with Resulting PK Profile

In an effort to better quantify the infusion, a 1mg/mL commercial formulation of naloxone was infused into the pig using the hollow microneedle POC device. Naloxone is a µ-opioid receptor competitive antagonist used primarily to combat overdose of drugs such as heroin. Typically administered intravenously for fast response, naloxone is only about 2% bioavailable when administered orally. Naloxone is well-absorbed but is nearly 90% removed during first pass. Literature review indicates that the half life of naloxone in human adults is 30-81 minutes and considerably longer (approx 3 hours) in children. Naloxone is excreted in the urine as metabolites.

Blood samples were collected from the ear vein of the pig before infusion and at specified time points up to 2 hours following infusion. The samples were prepared and analyzed to determine naloxone level in sera. For comparison, naive pigs were dosed with the same commercial naloxone formulation using either subcutaneous or intravenous injection. As with the intradermal infusion, blood samples were collected and analyzed for naloxone levels.

Three different animals were used for the study comparing the PK profiles generated after hollow microneedle infusion, subcutaneous injection and IV injection. The pigs weighed between 10-22 kg at the time of dosing and ranged in age from 1.5-3 months. A commercial formulation (1mg/mL) of naloxone hydrochloride (International Medication Systems, Ltd, So. El Monte, CA) was used for the infusion. Table IV shows the infusion profiles used with the naloxone administrations performed with the hollow microneedle device.

**Table IV. Summary of infusion conditions for naloxone infusion**

| **Total Volume** | **Max Rate** | **Infusion RateProfile in µL/min (time)** |
|---|---|---|
| 425µL | 30 (µL/min) | 10(5min), 20(7.5min), 30(duration) |
| 200µL | 75µL/min | 25(1min), 50(2min), 75(duration) |
| 330µL | 75µL/min | 10(1min), 25(2min), 50(5min), 75(duration) |

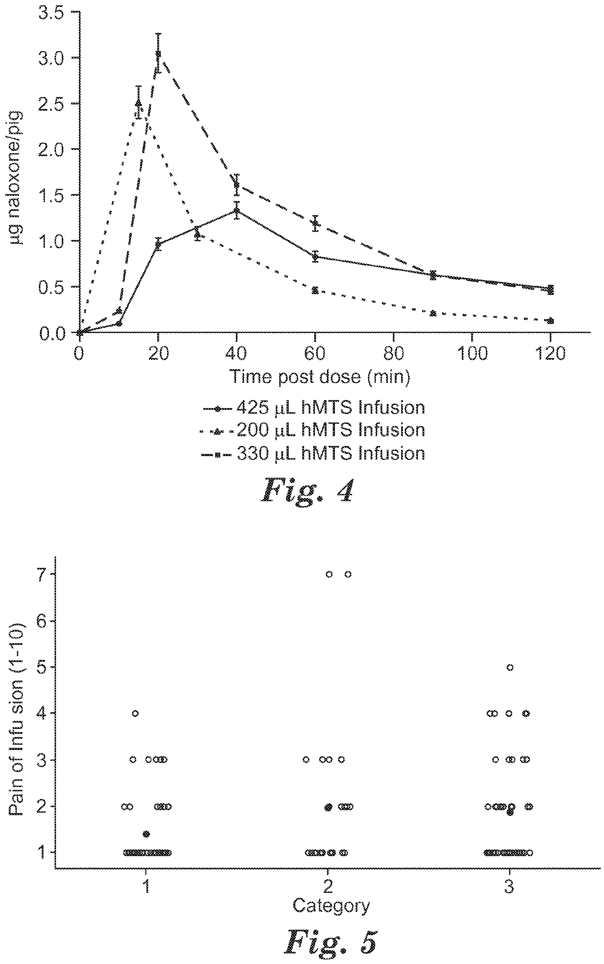

A comparative graph of naloxone blood levels versus time by delivery route is shown in Figure 4. Pigs were also administered naloxone via subcutaneous injection. These pigs were similar in weight and age to those administered naloxone via the hollow microneedle device. These results indicate comparable delivery of naloxone via the hollow microneedle and subcutaneous injection. Based on blood samples collected up to 2 hours after initiation of the infusion, the bioavailability for the naloxone administered by the hollow microneedle technology is estimated to be 107+/-35% of that resulting from subcutaneous administration.

### Example 3. Human Infusion Study with dextrose

Using the same apparatus described above, a demonstration of the high volume, high rate infusion was conducted on humans. During a human clinical trial, 28 subjects were administered 4-6 sequential hollow microneedle placebo infusions to their upper arms and/or upper legs. Back pressure was monitored continuously throughout the infusion. Using a 10-point pain scale (see Figure 4), each subject was asked to rate the pain associated with application and removal of the hollow microneedle patch; subjects were also asked to rate pain associated with infusion every 10 minutes during the infusion or at the end of infusion if the infusion ended in less than 10 minutes.

Figures 5, 9, 10 and 11 plot data involving pain based on the following pain scale.

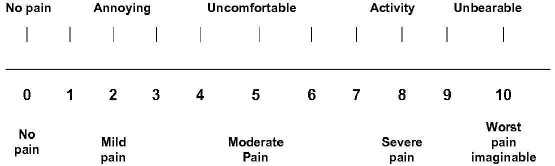

Of the 125 infusions initiated, 46 infusions equal to or greater than 750µL were administered. Different infusion rate profiles were used during the study, encompassing infusion rates from 10-433µL/min. There was no statistically significant difference between the subjects' perceived pain and the volume of the infusion. Table V summarizes, by category, highest infusion rates, infusion volume and maximum discomfort during infusion for those subjects receiving high volume (Category 3, >750µL) infusions.

**Table V. Summary of infusion parameters by category**

| | **# of Infusions** | **Avg Pain** | **Avg Back Pressure (psi)** | **Avg Vol µL** | **Highest Rate µL/min** |
|---|---|---|---|---|---|
| Category 1 (0-250µL) | 52 | 1.40+/-0.77 | 8.8+/-5.2 | 133+/-60 | 76+/-79 |
| Category 2 (250-750µL) | 27 | 1.96+/-1.66 | 13.5+/-5.0 | 427+/-148 | 90+/-73 |
| Category 3 (750-1000µL) | 46 | 1.83+/-1.12 | 13.37+/-4.20 | 970+/-65 | 126+/-93 |

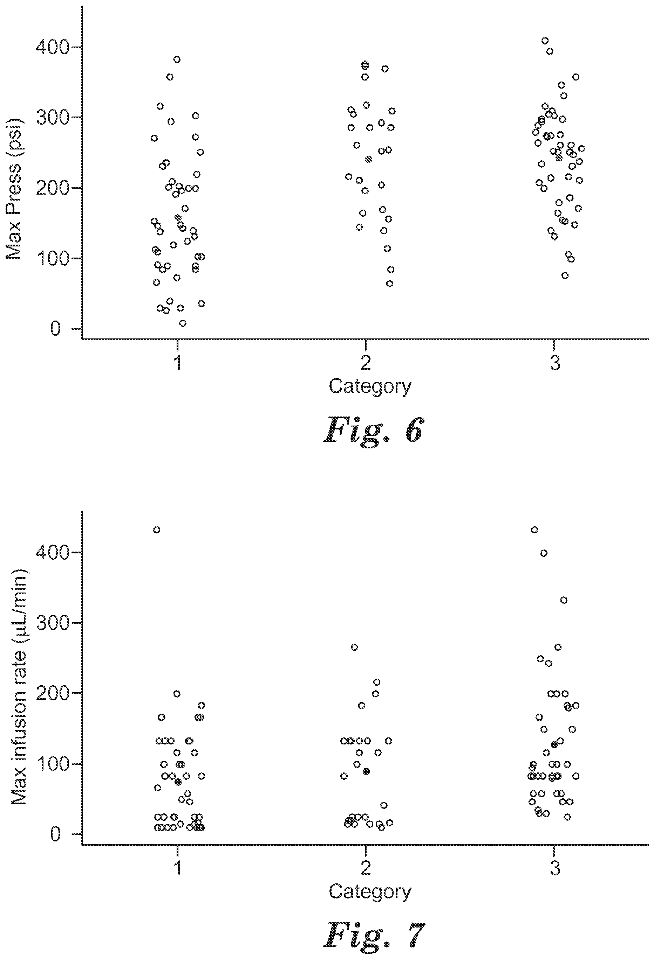

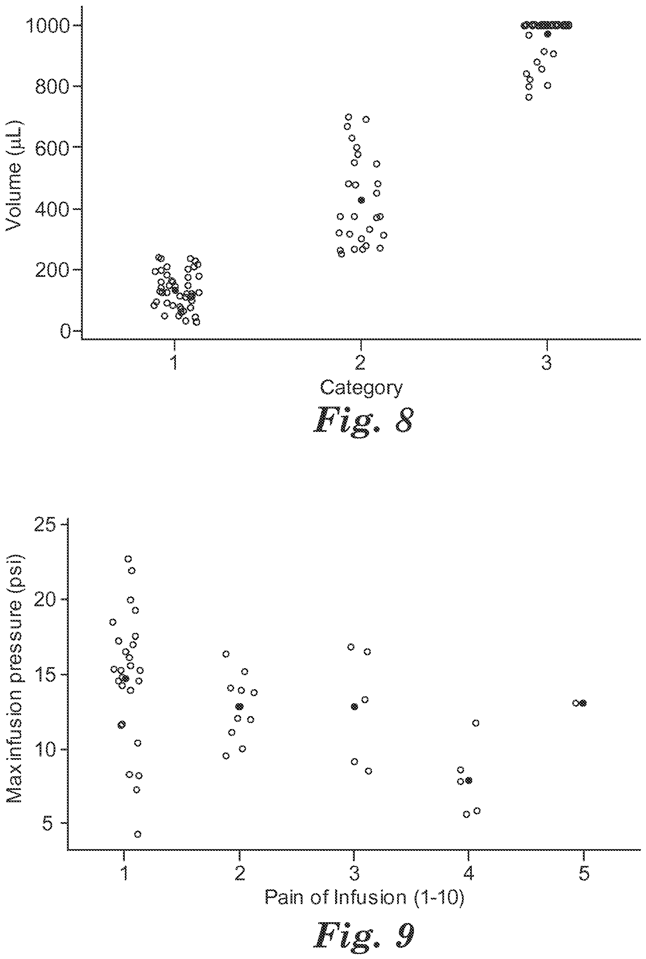

Figures 5-8 provide a distribution summary of infusion parameters sorted by category. Figure 5 plots pain of infusion versus Category. Figure 6 plots maximum infusion pressure versus Category. Figure 7 plots maximum infusion rate versus Category. Figure 8 plots infusion volume versus Category.
Table VI provides a summary of infusion parameters for all Category 3 infusions.

**Table V1. Summary of infusion parameters and pain scores for subjects receiving high volume infusions**

| **Subj ID** | **Site** | **End rate (µL/min)** | **Initial rate (mcL/min)** | **Max rate (µL/min)** | **Vol (µL)** | **MaxPress (psi)** | **Total Infusion Time (min)** | **Pain of Infusion** |
|---|---|---|---|---|---|---|---|---|
| 7 | LLT | 30 | 10 | 30 | 800 | 9.6 | | 2 |
| 7 | LUT | 35 | 10 | 35 | 1000 | 8.3 | | 1 |
| 7 | RUT | 46.7 | 10 | 46.7 | 908 | 7.3 | | 1 |
| 10 | LUT | 25 | | 25 | 767 | 5.6 | | 4 |
| 10 | LLT | 58.3 | 10 | 58.3 | 1001 | 11.8 | | 4 |
| 10 | RUT | 58.3 | | 58.3 | 1001 | 5.9 | | 4 |
| 11 | RLT | 30 | 10 | 30 | 804 | 16.9 | | 3 |
| 11 | LUT | 46.7 | 10 | 46.7 | 1000 | 12.9 | | 2 |
| 12 | LUT | 46.7 | 10 | 46.7 | 1001 | 7.8 | | 4 |
| 12 | LLT | 58.3 | 10 | 58.3 | 1001 | 8.6 | | 4 |
| 12 | RLT | 80 | 10 | 80 | 1001 | 13.1 | | 5 |
| 13 | LLT | 66.7 | 166.7 | 166.7 | 1000 | 13.3 | | 3 |
| 13 | RUT | 95 | 25 | 95 | 1000 | 9.2 | | 3 |
| 13 | LUT | 58.3 | 16.6 | 58.3 | 1000 | 8.5 | | 3 |
| 14 | RA | 58.3 | 16.6 | 58.3 | 858 | 14.0 | | 1 |
| 15 | LLT | 83.3 | 83.3 | 83.3 | 1000 | 14.0 | | 2 |
| 15 | RLT | 243.3 | 83.3 | 243.3 | 1000 | 10.0 | | 2 |
| 16 | LUT | 100 | 83.3 | 100 | 1002 | 13.8 | | 2 |
| 16 | LLT | 58.3 | 83.3 | 83.3 | 1001 | 15.3 | | 1 |
| 17 | RA | 50 | 83.3 | 83.3 | 1000 | 11.6 | | 1 |
| 17 | LT | 58.3 | 83.3 | 83.3 | 1000 | 14.1 | | 2 |
| 17 | RT | 58.3 | 83.3 | 83.3 | 1000 | 16.6 | | 3 |
| 18 | LUT | 83.3 | 83.3 | 83.3 | 1001 | 11.1 | | 2 |
| 18 | RUT | 100 | 83.3 | 100 | 1001 | 12.1 | | 2 |
| 19 | LLT | 83.3 | 83.3 | 83.3 | 1000 | 12.0 | | 2 |
| 19 | LUT | 100 | 83.3 | 100 | 823 | 14.3 | | 1 |
| 19 | RUT | 100 | 83.3 | 100 | 1000 | 4.3 | | 1 |
| 20 | LA | 66.7 | 83.3 | 83.3 | 1001 | 14.6 | | 1 |
| 20 | RA | 50 | 83.3 | 83.3 | 1001 | 14.6 | | 1 |
| 21 | LUT | 100 | 100 | 100 | 1000 | 15.2 | 0:10:11 | 2 |
| 23 | LLT | 200 | 100 | 200 | 840 | 16.4 | 0:05:30 | 2 |
| 22 | LLT | 166.7 | 100 | 166.7 | 1000 | 17.6 | 0:08:30 | 1 |
| 22 | LMT | 183.3 | 116.7 | 183.3 | 1001 | 19.3 | 0:07:12 | 1 |
| 24 | LUT | 266.7 | 66.7 | 266.7 | 1000 | 15.6 | 0:06:30 | 1 |
| 26 | LMT | 250 | 100 | 250 | 880 | 15.3 | 0:06:00 | 1 |
| 26 | LLT2 | 116.7 | 116.7 | 116.7 | 1002 | 14.8 | 0:10:50 | 1 |
| 26 | RLT | 100 | 133.3 | 133.3 | 1001 | 16.2 | 0:12:10 | 1 |
| 28 | RUT | 200 | 200 | 200 | 1000 | 17.3 | 0:07:06 | 1 |
| 28 | LUT | 150 | 400 | 400 | 913 | 16.6 | 0:07:58 | 1 |
| 28 | LLT | 100 | 433.3 | 433.3 | 1000 | 10.4 | 0:11:14 | 1 |
| 25 | RMT | 150 | 100 | 150 | 970 | 22.0 | 0:09:00 | 1 |
| 25 | RUT | 180 | 117 | 180 | 1000 | 20.0 | 0:08:48 | 1 |
| 27 | LUT | 117 | 150 | 150 | 1000 | 18.5 | 0:11:00 | 1 |
| 27 | RUT | 183 | 167 | 183 | 1000 | 15.4 | 0:07:00 | 1 |
| 27 | RLT | 200 | 200 | 200 | 1000 | 22.8 | 0:07:38 | 1 |
| 27 | LMT | 117 | 333 | 333 | 1000 | 17.0 | 0:10:17 | 1 |

| | | | | | | | | |
|---|---|---|---|---|---|---|---|---|
| Key to site: 1-L/R=left/right; 2-L/U/M=lower/upper/mid; 3-T/A=thigh/arm | | | | | | | | |

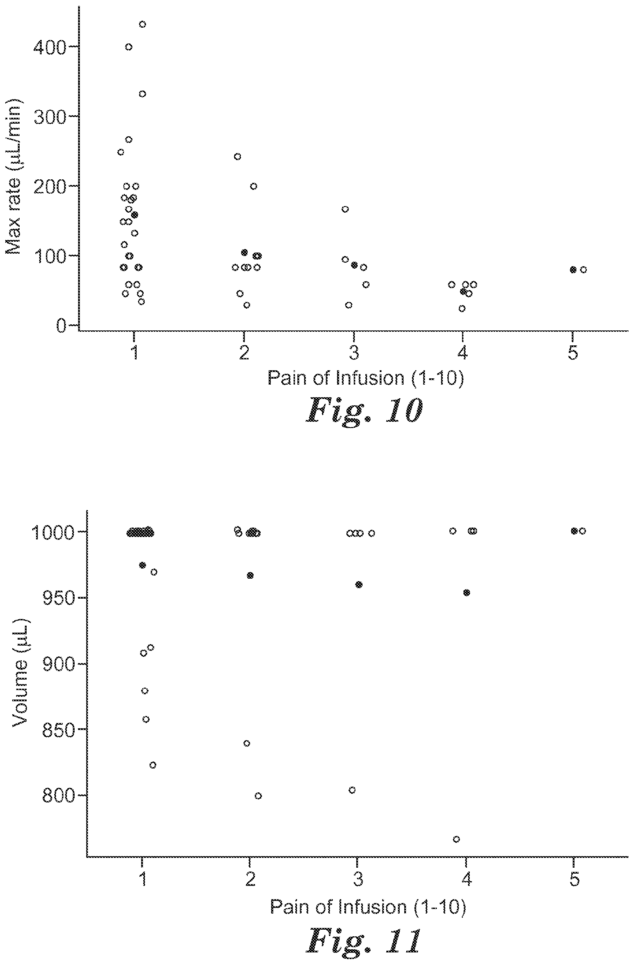

Figures 9-11 plot the relationships between infusion pain and various infusion parameters for Category 3 (750-1000µL) infusions only. Figure 9 plots pain of infusion versus maximum infusion pressure. Figure 10 plots pain of infusion versus maximum infusion rate. Figure 11 plots pain of infusion versus infusion volume.

It will be understood that various unforeseen modifications and alterations to this invention will become apparent to those skilled in the art without departing from the scope of this invention. It should be understood that this invention is not intended to be unduly limited by the illustrative embodiments and examples set forth herein and that such examples and embodiments are presented by way of example only with the scope of the invention intended to be limited only be the claims set forth herein as follows.

## Claims

1. A hollow microneedle array (11) for rapid, high-volume, intradermal infusion with minimal pain, the hollow microneedle array (11) comprising:
an array of 10 to 30 hollow microneedles (14) having a length of greater than 100um to less than 1mm.;
the hollow microneedle array (11) being adapted for pumping greater than 200 uL of fluid through the hollow microneedles at a rate of greater than 20 uL/min;
and being **characterized in that** the microneedles (14) are spaced an average of at least 2 mm apart from each other.

2. The hollow microneedle array (11) of claim 1, wherein the array has 13 to 20 microneedles (14).

3. The hollow microneedle array (11) of claims 1 or 2, wherein the hollow microneedles (14) have an average channel bore of 20 to 50 um² cross-sectional area.

4. The hollow microneedle array (11) of claim 1, wherein the microneedles (14) have a length of between 500 um and 750 um.

5. The hollow microneedle array (11) of claim 1, wherein the microneedles (14) have an exit hole located on a sidewall of each microneedle (14).

## Patentansprüche

1. Mikrohohlnadelanordnung (11) für eine schnelle, hochvolumige, intradermale Infusion unter minimalem Schmerz, wobei die Mikrohohlnadelanordnung (11) umfasst:
eine Anordnung aus 10 bis 30 Mikrohohlnadeln (14) mit einer Länge von mehr als 100 µm bis weniger als 1 mm;
wobei die Mikrohohlnadelanordnung (11) so konzipiert ist, dass sie mehr als 200 µl Flüssigkeit durch die Mikrohohlnadeln bei einer Flussrate von mehr als 20 µl/min pumpt;
und **dadurch gekennzeichnet, dass** die Mikronadeln (14) in einem durchschnittlichen Abstand von mindestens 2 mm voneinander angeordnet sind.

2. Mikrohohlnadelanordnung (11) nach Anspruch 1, wobei die Anordnung 13 bis 20 Mikronadeln (14) aufweist.

3. Mikrohohlnadelanordnung (11) nach den Ansprüchen 1 oder 2, wobei die Mikrohohlnadeln (14) eine durchschnittliche Kanalbohrungsquerschnittsfläche von 20 bis 50 µm² aufweisen.

4. Mikrohohlnadelanordnung (11) nach Anspruch 1, wobei die Mikronadeln (14) eine Länge zwischen 500 µm und 750 µm aufweisen.

5. Mikrohohlnadelanordnung (11) nach Anspruch 1, wobei die Mikronadeln (14) ein Austrittsloch, angeordnet an einer Seitenwand einer jeden Mikronadel (14), aufweisen.

## Revendications

1. Jeu de micro-aiguilles creuses (11) conçu pour une perfusion intradermique à haut volume, rapide avec un minimum de douleur, le jeu de micro-aiguilles creuses (11) comprenant :
un jeu de 10 à 30 micro-aiguilles creuses (14) ayant une longueur allant de plus de 100 µm à moins d'1 mm ;
le jeu de micro-aiguilles creuses (11) étant conçu pour pomper plus de 200 µL de fluide à travers les micro-aiguilles creuses, à un débit supérieur à 20 µL/min ;
et étant **caractérisé en ce que** les micro-aiguilles (14) sont espacées en moyenne d'au moins 2 mm l'une de l'autre.

2. Jeu de micro-aiguilles creuses (11) selon la revendication 1, dans lequel le jeu comporte de 13 à 20 micro-aiguilles (14).

3. Jeu de micro-aiguilles creuses (11) selon les revendications 1 ou 2, dans lequel les micro-aiguilles creuses (14) ont un alésage de canal moyen dont l'aire en coupe transversale va de 20 à 50 µm².

4. Jeu de micro-aiguilles creuses (11) selon la revendication 1, dans lequel les micro-aiguilles (14) ont une longueur comprise entre 500 µm et 750 µm.

5. Jeu de micro-aiguilles creuses (11) selon la revendication 1, dans lequel les micro-aiguilles (14) ont un trou de sortie situé sur une paroi latérale de chaque micro-aiguille (14).
